# EUROPEAN PATENT SPECIFICATION

(11) **EP 1 501 521 B1**
(45) Date of publication and mention of the grant of the patent: **02.05.2007**
(21) Application number: 02738611.9
(22) Date of filing: 23.04.2002
(51) Int. Cl.: A61K 31/7068, A61K 31/7072, A61K 9/22, A61P 31/18

(54) **LONG ACTING COMPOSITIONS COMPRISING ZIDOVUDINE AND/OR LAMIVUDINE**
LANG WIRKENDE ZUSAMMENSETZUNGEN ENTHALTEND ZIDOVUDINE UND/ODER LAMIVUDINE
COMPOSITIONS RETARD CONTENANT DE LA ZIDOVUDINE ET/OU DE LA LAMIVUDINE

(43) Date of publication of application: 02.02.2005
(73) Proprietor: Lupin Limited, Mumbai 400 098, Maharashtra (IN)
(72) Inventor: SEN, Himadri Lupin Research Park, District-Pune 411 042 Maharashtra (IN); JAYANTHI, Surya Kumar Lupin Research Park, District-Pune 411 042 Maharashtra (IN)
(74) Representative: Harrison Goddard Foote
(86) International application number: PCT/IN2002/000110
(87) International publication number: WO 2003/090762

(56) References cited:
- WO-A-00/15198
- WO-A-98/18477
- WO-A-99/55372
- US-A- 5 681 581

## Description

### FIELD OF THE INVENTION

The invention relates to a pharmaceutical composition of modified release tablets comprising lamivudine, zidovudine or combination of lamivudine and zidovudine or their pharmaceutically acceptable derivatives as the active ingredient(s), and a mixture of hydrophilic polymers selected from the group consisting of at least one hydroxypropyl methylcellulose, at least one sodium alginate and at least one guar gum as controlled release matrix and a pharmaceutically acceptable calcium salt as a matrix stabilizer. The composition also contains one or more of a water soluble and/or water dispersible diluent, wherein the quantities of the hydrophilic polymers, the pharmaceutically acceptable calcium salt and water soluble and/or water dispersible diluents are such that the therapeutically effective active ingredient(s) is/are released at a rate suitable for once daily administration of the pharmaceutical composition. Optionally, the tablets may be coated with a water soluble polymeric film coat.

### BACKGROUND OF THE INVENTION

Strategies for the treatment of human immunodeficiency virus (HIV) infection began changing with the recognition that viral replication occurs during the years preceding the development of clinical disease. Between the time of the initial infection and the development of clinical disease, T-lymphocyte CD4⁺ counts progressively decline as immune function becomes impaired.

The availability of an increasing number of antiretroviral agents and the rapid evolution of new information has introduced extraordinary complexity into the treatment of HIV-infected patients. The objectives of anti-AIDS (acquired immunodeficiency syndrome) therapy include maximal and durable suppression of viral load, restoration and/or preservation of immunologic function, improvement of quality of life, and reduction of HIV-related morbidity and mortality.

Antiretroviral drugs can be classified under three broad categories 1) Nucleoside Reverse Transcriptase Inhibitors (NRTIs) 2) Non-Nucleoside Reverse Transcriptase Inhibitors (NNRTIs) and 3) Protease Inhibitors (PIs). Some of the drugs widely used include zidovudine, lamivudine, stavudine and abacavir (NRTIs), nevirapine (NNRTI) and indinavir, ritonavir and saquinavir (PIs). For maximizing the benefits of antiretroviral therapy it is necessary to sequence the drugs rationally. In this context, three alternative regimens have been employed viz. a PI with two NRTIs, a NNRTI with two NRTIs or three NRTI regimen (see Gulick RM et al., *N. Engl. J. Med*., 1997, 337, 734; Montaner JSG et al., *JAMA*, 1998, 279, 930; Hogg RS et al., *JAMA*, 1998, 279,450).

While many compounds are known to be useful as pharmacologically active substances, some of them have relatively short biological half-life and need to be administered several times a day in order to achieve desired therapeutic effect. However, a decrease in the frequency of administration will not only reduce the burden on the patient but will also increase compliance and thus provide greater therapeutic effect. It can be achieved by controlling the release of active ingredients, so that the effective level is maintained in the blood for a prolonged period of time.

This has been primarily achieved by development of new drug delivery systems utilizing diverse techniques and principles. Amongst these, known in the art is one such delivery system, which employs hydrophilic polymers to produce sustained or modified release pharmaceutical compositions. For modified release solid dosage forms comprising a drug dispersed uniformly in hydrophilic polymers, release of the drug is controlled primarily by diffusion of the drug, or by surface erosion of the hydrophilic polymers into the surrounding medium, or by a combination of the two processes. Control of the rate of release can produce constant blood levels of the active ingredient that may result in reducing the frequency of administration, thereby improving patient compliance to the dosage regimen.

United States Patent No. 6,113,920 discloses a pharmaceutical composition comprising two active pharmaceutical ingredients namely lamivudine and zidovudine and a pharmaceutically acceptable glidant ingredient, selected from a group of colloidal silicon dioxide, microcrystalline cellulose, metallic stearates, calcium carbonate and combinations thereof, in the form of a film coated tablet. The composition contains lamivudine in an amount from 15 to 1500 mg per tablet and zidovudine in an amount from 30 to 1000 mg per tablet.

The PCT application WO 02/00168 discloses a pharmaceutical composition comprising of 20(S)-camptothecin, a derivative, an analog, a prodrug or a metabolite thereof in combination with one or more agents selected from the group consisting of nucleoside reverse transcriptase inhibitor, non-nucleoside reverse transcriptase inhibitor, protease inhibitor, fusion inhibitor and integrase inhibitor for the treatment of HIV infection.

The patent application EP 1166782 A3 discloses a composition comprising caffeic acid phenethyl ester, methyl caffeate, phenethyl dimethyl caffeate or 4-bromocinnamic acid phenethyl ester optionally in combination with other antiviral agents, such as a protease inhibitor, a nucleoside reverse transcriptase inhibitor, a non-nucleoside reverse transcriptase inhibitor, or an integrase inhibitor for treatment or prophylaxis of a disorder related to retrovirus infection or for the inhibition of retrovirus replication.

The United States Patent Application US 2001/0005712 A1 teaches a method of treating patients having AIDS or HIV infections by parenteral administration of Product R, a peptide-nucleic acid preparation, in a combination with one or more antiviral agents useful for treating AIDS or HIV infections including HIV protease inhibitors and nucleoside analogs.

The PCT application WO 01/10454 discloses a method of inhibiting or treating HIV infection by administration of a composition comprising a PEG-ASNase compound (a conjugate of asparaginase with polyethylene glycol) or a pharmaceutically acceptable salt thereof, and optionally at least one compound selected from the group consisting of protease inhibitor compounds, ribonucleotide reductase inhibitor compounds and HIV reverse transcriptase inhibitor compounds.

The PCT application WO 00/51641 discloses a pharmaceutical combination comprising antiviral dioxolane nucleoside and further therapeutic agent chosen from nucleoside analogs, non-nucleoside reverse transcriptase inhibitors, and protease inhibitors, which is useful for the treatment of viral infections.

The PCT application WO 00/18383 discloses antiviral combination of (S)-2-ethyl-7-fluoro-3-oxo-3,4-dihydro-2H-quinoxaline-1-carboxylic acid isopropyl ester and zidovudine and/or lamivudine.

The PCT application WO 00/00479 teaches a method of treating HIV infection by administration of 1,3-benzodiazepin-2-ones or 1,3-benzoxazepin-2-ones in combination with at least one compound selected from HIV reverse transcriptase inhibitors or HIV protease inhibitors.

The PCT application WO 99/25352 discloses a synergistic composition comprising HIV protease inhibitor compound N- (2 (R)-hydroxy-1(S)-indanyl)-2(R)-phenylmethyl-4-(S)-hydroxy-5-(1-(4-(2-benzo[b]furanyl-methyl)-2(S)-N'-(t-butylcarboxamido)-piperazinyl))-pentaneamide, zidovudine and lamivudine for prevention or treatment of HIV infection.

The PCT application WO 98/46238 teaches a method for treatment or prevention of a viral infection by administration of at least one biflavanoid and at least one antiviral compound. The list of biflavanoid includes robustaflavone, hinokiflavone, amentoflavone, agathisflavone etc. and the list of antiviral compound includes zidovudine, lamivudine, alyclovir, gancyclovir etc.

The PCT application WO 98/32442 teaches use of quinoxalines in triple combination of with protease inhibitors and reverse transcriptase inhibitors for treatment of AIDS and/or HIV infections.

The PCT application WO 96/23509 teaches a combination of the HIV protease inhibitor, indinavir, lamivudine and optionally a compound selected from zidovudine, dideoxyinosine and dideoxycytidine useful in the prevention or treatment of infection by HIV and the treatment of AIDS.

The PCT application WO 96/01110 teaches a combination of zidovudine, lamivudine and loviride for the treatment of HIV infections and AIDS.

To maintain blood levels of the anti HIV chemotherapeutic agents on a continuous regular basis for a long time, frequent dosing is required. Such frequent dosing cause problems in ensuring patient compliance.

The relevant prior art methods, which teach adaptation of diverse delivery systems for sustained release of the actives, are as follows.

United States Patent No. 6,210,712 relates to an extended release osmotic dosage form comprising a drug, a pharmaceutically acceptable carrier in a core, coated by a bilayer coat, the interior coat consists of ethylcellulose and hydroxyalkyl cellulose and it is shielded by a second/exterior coat of poly(cellulose acylate). The carrier can be a hydrogel and representative polymer hydrogels comprise a maltodextrin polymer, a poly(alkylene oxide), an alkali carboxyalkylcellulose, and a copoylmer of ethylene-acrylic acid.

The United States Patent No. 6,287,599 relates to a sustained release composition comprising a non-pH-dependent sustained release agent in an amount from 5-50 wt %, preferably from 10-30 wt %; and a pH-dependent agent in an amount from 0.5-40 wt %, preferably from 1-20 wt %, such that it increases the rate of release of at least one pH-dependent active at a pH in excess of 5.5. The composition is especially suitable for guanfacine hydrochloride, guanadrel sulfate, reserpine, anagrelide hydrochloride, porpanolol, metoprolol, erythromycin, clonidine, chlorpheniramine, diltiazem and scopolamine.

United States Patent No. 4,917,900 discloses a pharmaceutical formulation for oral administration in which discrete units comprising zidovudine are each provided with a controlled release coating consisting of alkyl esters of acrylic and methacrylic acids and ethylcellulose in a weight ratio of 1:3 to 3:1. These spheroids contain at least 80% of zidovudine and microcrystalline cellulose and mannitol as the core-forming agent.

United States Patent No. 6,083,532 discloses a sustained release tablet comprising a drug to be released at a controlled rate and a sustained release formulation comprising at least three different types of polymers including a pH dependent gelling polymer, a pH independent gelling polymer and an enteric polymer wherein pH dependent gelling polymer comprises at least one of an alginate, a carboxyvinyl polymer, or a salt of a carboxymethyl cellulose; pH independent gelling polymer comprises at least one of a hydroxypropyl methyl cellulose, a hydroxypropyl ethyl cellulose, a hydroxypropyl cellulose, a hydroxyethyl cellulose, a methyl cellulose, a guar gum or a polyethylene oxide; and enteric polymer comprises at least one of a polyacrylate material, a cellulose acetate phthalate, a cellulose phthalate hydroxypropyl methyl ether, a polyvinyl acetate phthalate, a hydroxypropyl methyl cellulose acetate succinate, a cellulose acetate trimellitate, or a shellac.

The PCT application WO 94/03471 teaches a controlled release zidovudine formulation such that a therapeutic level of zidovudine (AZT) is maintained but the side effects caused by its catabolite, 3'-amino-3-deoxythymidine (AMT) are substantially reduced. This is achieved by a formulation, which releases AZT for intracellular phosphorylation at a rate that does not saturate the uridine diphosphoglucuronyltransferase pathway in converting AZT to glucuronide. The controlled release is achieved by coating the drug/granules containing the drug, with an acid resistant (e.g. polyvinyl acetate phthalate, hydroxypropylmethyl cellulose phthalate and Eudragit) and/or hydrophobic polymer (e.g. ethyl cellulose, zein, certain methacrylate copolymers). These coated granules are formed by providing one or more coatings serially on the granules. These polymer-coated granules are then incorporated into a lipid hydrogel polymer matrix.

Patent application WO 00/15198 teaches controlled delivery pharmaceutical composition having temporal and spatial control, comprising a drug, a gas-generating component, a swelling agent, a viscolyzing agent, and optionally a gel forming polymer. The viscolyzing agent initially and the gel forming polymer thereafter form a hydrated gel matrix which entraps the gas, causing the tablet to float so that it is retained in the stomach thereby providing spatial control and at the same time resulting in sustained release of the drug providing temporal control. The combination of gas generating component, swelling agent and viscolyzing agent results in the controlled drug delivery system. Thus all these components are essential for achieving the temporal and spatial control. A preferred once daily ciprofloxacin formulation comprising 69.9 % ciprofloxacin base, 0.34 % sodium alginate, 1.03 % xanthan gum, 13.7 % sodium bicarbonate, 12.1 % cross-linked polyvinylpyrrolidone and optionally other excipients is disclosed.

United States Patent No. US 6,267,986 B1 teaches preparation of a controlled release pseudoephdrine composition in combination with an antihistamine comprising two discrete zones. The first discrete zone comprises pseudoephdrine, one or more hydrophilic polymers in the range of 0.1 to 90 %, preferably 20-50 % by weight, a salt of a polyuronic acid and a pharmaceutically acceptable salt of a group II metal ion and the second discrete zone comprises an antihistamine.

The PCT Application No. PCT/IN01/00204 relates to an improved, stable pharmaceutical composition of modified release tablets comprising a betalactam antibiotic or their pharmaceutically acceptable hydrates, salts or esters as the active ingredient, and a mixture of hydrophilic polymers selected from the group consisting of at least one sodium alginate and at least one xanthan gum and a pharmaceutically acceptable calcium salt and optionally a hydroxypropyl methylcellulose as controlled release matrix.

Combination antiretroviral therapy is now used to maintain viral suppression and prevent the emergence of viral resistance to antiretroviral agents. It reduces the risk of HIV disease progression and death. The current recommendations for initiating antiretroviral treatment suggest that antiretroviral therapy be considered in any patient with a viral load higher than 5,000 to 20,000 copies per mL, regardless of the CD4⁺ count. This comes as an addition to previous recommendations to institute therapy in symptomatic patients and in patients with CD4⁺ counts of less than 500 per mm³. Effective combination therapy should decrease a patient's viral load by at least 1 log (10-fold) after three to four weeks of treatment. The current therapeutic goal is to decrease viral load as much as possible, ideally to undetectable levels, four to six months after initiation of a new regimen (Maenza J et al., *Am. Fam. Physician*., June 1998, 57, 2789).

The antiretroviral agents now licensed in the United States for use in combination therapy include five nucleoside analog reverse transcriptase inhibitors (zidovudine, didanosine, zalcitabine, stavudine and lamivudine), two nonnucleoside reverse transcriptase inhibitors (delavirdine and nevirapine) and four protease inhibitors (saquinavir, ritonavir, indinavir and nelfinavir). Antiretroviral agents are rapidly being developed and approved, so physicians must make increasingly complex treatment decisions about medications having different dosage regimen.

As combinations of number of drugs having different dosage schedule need to be taken every day, for e.g. 150 mg lamivudine twice daily, 300 mg zidovudine twice daily, 800 mg indinavir every 8 hours etc., this could create patient compliance problem. This problem may be simplified if the drugs can be delivered once daily. It follows that such a once daily dosage regimen will improve patient compliance especially in view of long treatment duration.

There is a need for a simple, easy to make and cost effective controlled release composition of lamivudine and/or zidovudine, which can be administered once daily. The present invention provides the controlled release compositions of lamivudine, zidovudine or combination of lamivudine with zidovudine as active ingredient(s), which are efficacious, easy to make and cost-effective, suitable for once daily administration.

The controlled release compositions of the present invention can be formulated with lamivudine, zidovudine or combination of lamivudine with zidovudine as an active ingredient(s) such that the active ingredient(s) is maintained in the blood at therapeutically effective level for 24 hr resulting in once-daily administration of the composition thereby improving patient compliance to the dosage regimen. This has been achieved by controlling the release profile of the active ingredient(s) from the controlled release matrix, which in turn, can prolong blood levels over extended period of time.

The conventional dose of lamivudine is 150 mg twice daily. It has an oral bioavailability of 86 % and a serum half-life of 3-6 hours. The conventional dose of zidovudine is 300 mg twice daily and its oral bioavailability is 60 % with a serum half-life of 1.1 hours. There is lot of difference in the solubility profiles of lamivudine and zidovudine in various media such as water, 0.1 N HCl, pH 4.5 acetate buffer, pH 6.8 phosphate buffer etc.

The present invention gives the advantage of taking into account the different solubilities and half-lives of the drugs to achieve desired release profile for once a day administration of a composition containing lamivudine in combination with zidovudine. This is achieved by controlling the *in vitro* release of the actives in such a way that lamivudine with a longer half-life compared to zidovudine, is released faster and zidovudine with a shorter half-life compared to lamivudine, is released slowly.

The anti-AIDS drugs come under high dosing/high frequency category and extended release drug delivery systems have not been reported in reducing the frequency of dosing. The *in-vitro* intervention where the release profile of the active ingredient(s) from the delivery system is controlled by the combination of specific polymers, a pharmaceutically acceptable calcium salt, present in the matrix and their concentration thereby allowing prolonged blood levels over extended period of time, is employed to achieve long acting compositions containing lamivudine, zidovudine or combination of lamivudine with zidovudine that could be administered once daily.

Thus the object of the present invention is to provide a long acting pharmaceutical composition containing lamivudine, zidovudine, combination of lamivudine with zidovudine or pharmaceutically acceptable derivatives thereof as active ingredient(s) in a modified release matrix formulation, which is designed such that the resulting composition maintains the blood levels of the active ingredient(s) such that it is suitable for once daily administration.

### SUMMARY OF THE INVENTION

The present invention is directed to a long acting pharmaceutical composition of modified release tablets comprising lamivudine, zidovudine, combination of lamivudine with zidovudine or pharmaceutically acceptable derivatives thereof as active ingredient(s), and a mixture of hydrophilic polymers selected from the group consisting of at least one hydroxypropyl methylcellulose, at least one sodium alginate and at least one guar gum as controlled release matrix and a pharmaceutically acceptable calcium salt as a matrix stabilizer. The composition also contains one or more water soluble and/or water dispersible diluents, wherein the quantities of the hydrophilic polymers, the pharmaceutically acceptable calcium salt and water soluble and/or water dispersible diluents are such that the therapeutically effective active ingredients are released at a rate suitable for once daily administration of the pharmaceutical composition. The tablets may be coated with a water soluble polymeric film coat.

The effective therapeutic dose of the active ingredient(s) that can be administered by compositions of present invention include 300 mg of lamivudine, 600 mg of zidovudine or combination of 300 mg lamivudine with 600 mg zidovudine once daily.

Thus, according to a first preferred embodiment of the present invention, there is provided a pharmaceutical composition for controlled release of active ingredient comprising:
i. 4% to 50% by wt. of lamivudine or a pharmaceutically acceptable derivative thereof as the first active ingredient;
ii. 8% to 80% by wt. of zidovudine or a pharmaceutically acceptable derivative thereof as the second active ingredient;
iii. a controlled release matrix comprising a mixture of hydrophilic polymers consisting of 2% to 12% of at least one hydroxypropyl methylcellulose, 0.5% to 10% by wt. of at least one sodium alginate and 0.1 to 10% by wt. of at least one guar gum; and
iv. a pharmaceutically acceptable calcium salt.

A further preferred embodiment of the invention provides a pharmaceutical composition for controlled release of an active ingredient, said composition comprising:
i. an active ingredient selected from amongst lamivudine, zidovudine and combinations of lamivudine and zidovudine or their derivatives;
ii. a controlled release matrix comprising a mixture of hydrophilic polymers consisting of 2% to 15% by weight of at least one hydroxypropyl methylcellulose, 0.5 to 12% by wt. of at least one sodium alginate and 0.1 to 10% by wt. of at least one guar gum;
iii. a pharmaceutically acceptable calcium salt.

According to a still further preferred embodiment of the invention, there is provided a pharmaceutical composition for controlled release of an active ingredient, said composition comprising an active ingredient, selected from amongst lamivudine, zidovudine or their pharmaceutically acceptable derivatives, and a control release matrix comprising a mixture of hydrophilic polymers consisting of hydroxypropyl methylcellulose, sodium alginate and guar gum and a pharmaceutically acceptable calcium salt wherein said composition comprises 4% to 80% by weight of said active ingredient, and 1% to 25% by weight of said mixture of hydrophilic polymers, wherein said mixture of hydrophilic polymers consists of 0.5% to 12% by weight of sodium alginate, 0.1% to 14% by weight of guar gum, 2% to 15% by weight of hydroxypropyl methylcellulose and 0.1% to 1.8% by weight of said pharmaceutically acceptable calcium salt.

### BRIEF DESCRIPTION OF THE DRAWINGS

Figure 1 is a graph illustrating the dissolution profile of the actives when two polymers, hydroxypropyl methylcellulose (HPMC) & sodium alginate in comparison to three polymers, HPMC, sodium alginate and guar gum are used.
Figure 2 is a graph illustrating the release over time of lamivudine and zidovudine into the bloodstream for a composition of the present invention containing lamivudine in combination with zidovudine.
Figure 3 is a graph illustrating the release over time of zidovudine into the bloodstream for a composition of the present invention containing zidovudine in comparison to a reference conventional formulation of zidovudine.

### DETAILED DESCRIPTION OF THE INVENTION

The composition of this invention is in the form of a matrix tablet comprising the active ingredient(s), hydrophilic polymers, a pharmaceutically acceptable calcium salt, water soluble and/or water dispersible diluents, pharmaceutically acceptable tablet excipients for controlling the release of active ingredient(s).

Examples of the pharmaceutically acceptable calcium salts useful in the present invention are calcium sulphate, calcium chloride, calcium carbonate or calcium phosphate. The preferred pharmaceutically acceptable calcium salt is calcium sulphate.

Examples of other anti AIDS drug which may be used include stavudine, zalcitabine, abacavir, nevirapine, delavirdine, efavirenz, ritonavir, saquinavir, indinavir, nelfinavir, amprenavir, pharmaceutically acceptable hydrates, salts or esters thereof and combinations thereof.

Hydroxypropyl methylcellulose when used as matrix forming agent in sustained release tablets, is uniformly incorporated throughout the matrix tablet. Upon contact with water, the outer tablet skin is partially hydrated forming a gel layer. The rate of diffusion of actives out of the gel layer and the rate of erosion determines the overall tablet dissolution and drug delivery rates.

Guar gum when used as matrix forming agent in sustained release tablets has an optimum rate of hydration above pH 7.5 and it takes 2 to 4 hours to develop maximum viscosity and has maximum stability between pH 4 to 10.5.

Sodium alginate is a water soluble salt of alginic acid. Sodium alginate is insoluble below pH 3 and soluble above pH 3. The matrix formed by sodium alginate releases the drug slowly below pH 3 and shows a faster release rate above pH 3. Thus, when it is used along with guar gum to form the matrix it reduces the initial bursting effect and in the later stages, acts as channeling agent to increase the release rate of the drug.

These three polymers when used in appropriate concentrations along with a pharmaceutically acceptable calcium salt as a matrix stabilizer provides the desired release profile, when the delivery system travels through the GIT, having varying pH gradients. This is possible as hydroxypropyl methylcellulose and sodium alginate retains integrity of the tablet and reduces initial bursting effect and this property is later maintained by guar gum. Surprisingly, the polymers in appropriate combinations are not only effective compared with commonly used polymers, but works at low concentrations also.

We have conducted trials using different polymers such as hydroxypropyl methylcellulose, sodium alginate, xanthan gum and guar gum and various percentages of these polymers in combination. Some of the trials were also conducted by deleting one of these polymers.

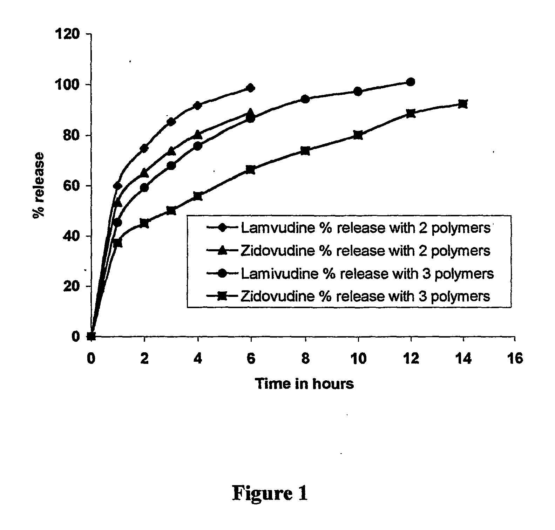

The results obtained when combination of two polymers i.e. 4 % hydroxypropyl methylcellulose and 2 % sodium alginate or combination of three polymers i.e. 4 % hydroxypropyl methylcellulose, 2 % sodium alginate and 2 % guar gum is employed are discussed in Table 1 and graphically represented in Fig.1. The use of only hydroxypropyl methylcellulose and sodium alginate was observed to release 90 % of the drugs in only 5 to 6 hours. The matrix integrity was also not retained for a long time, which resulted in a rapid initial surface erosion and faster release of the drugs.

The combination of hydroxypropyl methylcellulose, sodium alginate and guar gum forms an integrated matrix, as hydroxypropyl methylcellulose and sodium alginate reduce the initial bursting effect and in later stages act as channeling agent for the release of the drug. Guar gum after complete hydration retards the drug release at a later stage. The combinations of these polymers complement each other such that it overcomes the deficiencies associated with their use when used alone or in concentrations other than indicated in the invention.

**Table 1- % Release of the actives**

| **Duration in hr** | **Conventional (without polymers)** | | **Hydroxypropyl methylcellulose and sodium alginate** | | **Hydroxypropyl methylcellulose, sodium alginate and guar gum** | |
|---|---|---|---|---|---|---|
| | **% L^{a}** | **% Z^{b}** | **% L^{a}** | **% Z^{b}** | **% L^{a}** | **% Z^{b}** |
| 1 | 100 | 100 | 59.9 | 53.4 | 45.3 | 37.2 |
| 2 | | | 74.7 | 65.0 | 59.1 | 45.0 |
| 3 | | | 85.2 | 73.8 | 67.8 | 50.1 |
| 4 | | | 91.8 | 80.3 | 75.7 | 55.8 |
| 6 | | | 98.6 | 88.8 | 86.5 | 65.2 |
| 8 | | | | | 94.2 | 73.8 |
| 10 | | | | | 97.3 | 80.0 |
| 12 | | | | | 101.0 | 88.5 |
| 14 | | | | | | 92.3 |

| | | | | | | |
|---|---|---|---|---|---|---|
| ^{a}Percent lamivudine released ^{b}Percent zidovudine released | | | | | | |

According to the present invention, the pharmaceutical composition may contain a mixture of hydrophilic polymers of different viscosity grades selected from the group consisting of hydroxypropyl methylcellulose, sodium alginate and guar gum.

For the purpose of this patent application, hydroxypropyl methylcellulose may be characterized by their viscosities in a 2% w/v aqueous solution as low viscosity (about 100 cPs), medium viscosity (about 4000 to about 15000 cPs) and high viscosity (about 100,000 cPs), sodium alginate may be characterized by their viscosities in a 1 % w/v aqueous solution as low viscosity (about 75 to about 150 cPs), medium viscosity (about 200 to about 400 cPs) and high viscosity (about 600 to about 1000 cPs); and guar gum may be characterized by their viscosities in a 1% w/v aqueous solution as low viscosity (about 500 - 750 cPs), medium viscosity (about 2800 - 4200 cPs) and high viscosity (about 4800 - 5500 cPs).

Preferably sodium alginate used in the present invention is of medium or high viscosity, hydroxypropyl methylcellulose used is of medium or high viscosity and guar gum used is of medium or high viscosity. Low viscosity grade of hydroxypropyl methylcellulose, sodium alginate and guar gum can also be used but preferably medium or high viscosity grade polymers are used to utilize minimum possible concentrations to achieve the desired profiles, without compromising on the integrity of the matrix.

In the present invention sodium alginate having viscosity about or greater than 200 cPs, manufactured by ISP, USA, for example available under the brand name Keltone® HVCR; guar gum having viscosity greater than 4000 cPs, manufactured by Rhodia, USA, for example available under the brand name Meypro-Guar CSAA M-175 and hydroxypropyl methylcellulose having viscosity greater than 4000 cPs manufactured by DOW Chemicals, USA, for example available under the brand name Methocel^{™} K15 MP have been employed.

In a preferred embodiment of the present invention, the pharmaceutical composition comprises
(i) lamivudine or a pharmaceutically acceptable derivative thereof as the first active ingredient,
(ii) zidovudine or a pharmaceutically acceptable derivative thereof as the second active ingredient,
(iii) a mixture of hydrophilic polymers, said hydrophilic polymers being selected from the group consisting of at least one hydroxypropyl methylcellulose, at least one sodium alginate and at least one guar gum, and
(iv) a pharmaceutically acceptable calcium salt,
wherein said composition comprises about 4 % to about 50 % by weight of lamivudine or a pharmaceutically acceptable derivative thereof and about 8 % to about 80 % by weight of zidovudine or a pharmaceutically acceptable derivative thereof and about 1 % to about 20 % by weight of said mixture of hydrophilic polymers, wherein said mixture of hydrophilic polymers comprises about 0.5 % to about 10 % by weight of sodium alginate, about 0.1 % to about 10 % by weight of guar gum, about 2 % to about 12 % by weight of hydroxypropyl methylcellulose and about 0.1 % to about 1.8 % by weight of the calcium salt.

In a more preferred embodiment of the present invention, the pharmaceutical composition comprises
(i) lamivudine or a pharmaceutically acceptable derivative thereof as the first active ingredient,
(ii) zidovudine or a pharmaceutically acceptable derivative thereof as the second active ingredient,
(iii) a mixture of hydrophilic polymers, said hydrophilic polymers being selected from the group consisting of at least one hydroxypropyl methylcellulose, at least one sodium alginate and at least one guar gum, and
(iv) calcium sulphate,
wherein said composition comprises about 4 % to about 50 % by weight of lamivudine or a pharmaceutically acceptable derivative thereof and about 8 % to about 80 % by weight of zidovudine or a pharmaceutically acceptable derivative thereof and about 1 % to about 18 % by weight of said mixture of hydrophilic polymers, wherein said mixture of hydrophilic polymers comprises about 0.5 % to about 8 % by weight of a medium or high viscosity grade sodium alginate, about 0.3 % to about 8 % by weight of a medium or high viscosity grade guar gum, about 2 % to about 10 % by weight of a medium or high viscosity grade hydroxypropyl methylcellulose and about 0.1 % to about 1.5 % by weight of calcium sulphate.

In one more preferred embodiment of the present invention, the pharmaceutical composition comprises
(i) lamivudine or a pharmaceutically acceptable derivative thereof as the first active ingredient,
(ii) zidovudine or a pharmaceutically acceptable derivative thereof as the second active ingredient,
(iii) a mixture of hydrophilic polymers, said hydrophilic polymers being selected from the group consisting of at least one hydroxypropyl methylcellulose, at least one sodium alginate and at least one guar gum, and
(iv) calcium sulphate,
wherein said composition comprises about 4 % to about 50 % by weight of lamivudine or a pharmaceutically acceptable derivative thereof and about 8 % to about 80 % by weight of zidovudine or a pharmaceutically acceptable derivative thereof and about 1 % to about 15 % by weight of said mixture of hydrophilic polymers, wherein said mixture of hydrophilic polymers comprises about 1 % to about 6 % by weight of a medium or high viscosity grade sodium alginate, about 0.5 % to about 6 % by weight of a medium or high viscosity grade guar gum, about 3 % to about 8 % by weight of a medium or high viscosity grade hydroxypropyl methylcellulose and about 0.1 % to about 1.2 % by weight of calcium sulphate.

In another preferred embodiment of the present invention, the pharmaceutical composition comprises lamivudine, zidovudine or their pharmaceutically acceptable derivative as the active ingredient, and a mixture of hydrophilic polymers, said hydrophilic polymers being selected from the group consisting of at least one hydroxypropyl methylcellulose, at least one sodium alginate and at least one guar gum and a pharmaceutically acceptable calcium salt wherein said composition comprises about 4 % to about 80 % by weight of said active ingredient, and about 1 % to about 25 % by weight of said mixture of hydrophilic polymers, wherein said mixture of hydrophilic polymers comprises about 0.5 % to about 12 % by weight of sodium alginate, about 0.5 % to about 8 % by weight of guar gum, about 2 % to about 15 % by weight of hydroxypropyl methylcellulose and about 0.1 % to about 1.8 % by weight of a pharmaceutically acceptable calcium salt.

In still preferred embodiment of the present invention, the pharmaceutical composition comprises lamivudine, zidovudine or their pharmaceutically acceptable derivative as the active ingredient, and a mixture of hydrophilic polymers, said hydrophilic polymers being selected from the group consisting of at least one hydroxypropyl methylcellulose, at least one sodium alginate and at least one guar gum and a pharmaceutically acceptable calcium salt wherein said composition comprises about 4 % to about 80 % by weight of said active ingredient, and about 1 % to about 22 % by weight of said mixture of hydrophilic polymers, wherein said mixture of hydrophilic polymers comprises about 1 % to about 10 % by weight of a medium or high viscosity grade sodium alginate, about 0.5 % to about 6 % by weight of a medium or high viscosity grade guar gum, about 3 % to about 12 % by weight of a medium or high viscosity grade hydroxypropyl methylcellulose and about 0.1 % to about 1.5 % by weight of calcium sulphate.

In one more preferred embodiment of the present invention, the pharmaceutical composition comprises lamivudine, zidovudine or their pharmaceutically acceptable derivative as the active ingredient, and a mixture of hydrophilic polymers, said hydrophilic polymers being selected from the group consisting of at least one hydroxypropyl methylcellulose, at least one sodium alginate and at least one guar gum and a pharmaceutically acceptable calcium salt wherein said composition comprises about 4 % to about 80 % by weight of said active ingredient, and about 1 % to about 20 % by weight of said mixture of hydrophilic polymers, wherein said mixture of hydrophilic polymers comprises about 2 % to about 8 % by weight of a medium or high viscosity grade sodium alginate, about 1 % to about 4 % by weight of a medium or high viscosity grade guar gum, about 5 % to about 10 % by weight of a medium or high viscosity grade hydroxypropyl methylcellulose and about 0.1 % to about 1.2 % by weight of calcium sulphate.

The pharmaceutical composition of the present invention may contain one or more of pharmaceutically acceptable diluents. These diluents may be water soluble or water dispersible. Examples of water soluble diluents that may be used in the present invention include lactose, polyvinyl pyrrolidone and the like. Water dispersible diluent refers to insoluble pharmaceutical excipients, which disperse readily in water such as microcrystalline cellulose, starch, pre-gelatinized starch, dicalcium phosphate, magnesium aluminum silicates and the like.

Preferably the water soluble and/or dispersible diluent may be present in an amount from about 1 % to about 28 % by weight of the total weight of the composition containing lamivudine and zidovudine as active ingredients. In one preferred embodiment the water dispersible diluent is microcrystalline cellulose present in amount from about 5 % to about 20 % by weight of the composition. In another preferred embodiment the water dispersible diluent is dicalcium phosphate present in amount from about 1 % to about 5 % by weight of the composition.

Preferably the water soluble and/or dispersible diluent may be present in an amount from about 1 % to about 55 % by weight of the total weight of the composition containing lamivudine or zidovudine as an active ingredient. In one preferred embodiment the water dispersible diluent is microcrystalline cellulose present in amount from about 5 % to about 30 % by weight of the composition. In another preferred embodiment the water dispersible diluent is dicalcium phosphate present in amount from about 2 % to about 10 % by weight of the composition.

According to present invention the pharmaceutical composition may also contain tablet lubricants, at a concentration in the range of about 0.2 % to 3 % by weight of the composition. The lubricants that may be used include talc, stearic acid, magnesium stearate, calcium stearate, zinc stearate, hydrogenated vegetable oil and the like. Preferably the lubricant is selected from talc, stearic acid and magnesium stearate.

The pharmaceutical composition of the present invention may be prepared by procedures well known to formulation chemists. The method of manufacturing can affect the release characteristics of the composition. The active ingredients or their pharmaceutically acceptable derivatives; the hydrophilic polymers hydroxypropyl methylcellulose, sodium alginate, guar gum and the matrix stabilizer, pharmaceutically acceptable calcium salt such as calcium sulphate, one or more water soluble or water dispersible diluents are mixed together with lubricants and the blend is directly compressed into slugs by compaction followed by sieving and the granules obtained are lubricated and compressed into tablets. The active ingredients can be given as controlled release tablets for once a day administration The fines incorporated in the blend of active granules form about 15 % to about 35 %, preferably from about 12 % to about 25 % by weight.

The tablets may be optionally coated with a thin film of water soluble polymer. The tablets may be coated to a weight build-up of about 1 % to about 4 % by weight, preferably from about 2 % to about 3 % by weight. The preferred polymer for film coating of the tablets is hydroxypropyl methylcellulose E-5.

For the purposes of this patent application, fines denote the particles having size less than 250 microns.

The above-mentioned process has the advantage over its granulation by aqueous or non-aqueous vehicle used conventionally. The polymers used in the composition of present invention, hydroxypropyl methylcellulose, guar gum and sodium alginate are unstable above 60°-70° C. As the process is devoid of use of any solvents the potential problem of limiting the residual organic solvent is eliminated.

The modified release matrix formulation prepared according to the present invention is not a mere admixture but has properties different from the sum total of the properties of its ingredients.

The modified release matrix formulation prepared according to present invention can be administered once daily. The effective therapeutic dose of the active that can be administered once daily by compositions of present invention include 300 mg of lamivudine or 600 mg of zidovudine or 300 mg of lamivudine in combination with 600 mg of zidovudine.

A study was conducted for bioavailability in accordance with the composition of this invention. Six healthy male volunteers were selected for the study in which each volunteer was administered a combination tablet of lamivudine (300 mg) and zidovudine (600 mg) prepared according to the present invention, with 240 ml of water. The volunteers were given high fat standard breakfast before taking the tablet Blood samples were taken at regular intervals for a period of 24 hours.

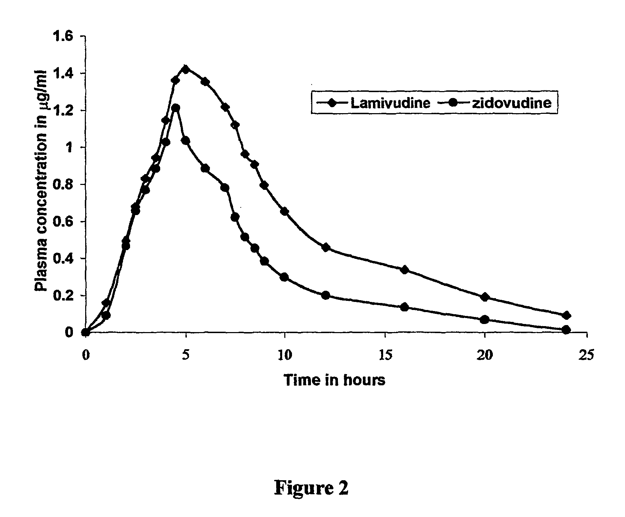

Figure 2 shows a plot of blood level concentration of lamivudine and zidovudine for the controlled release composition containing 300 mg lamivudine and 600 mg zidovudine prepared according to the present invention. The blood levels are achieved within 1 hour and detectable blood levels are present for 20 hours, clearly indicating that it can be used as once daily composition.

A randomized cross over bioavailability study was conducted for a zidovudine test formulation in accordance with the composition of the present invention containing 600 mg zidovudine and a reference conventional formulation containing 300 mg zidovudine, being marketed as ZIDOVIR^{™} 300 (Cipla). Six healthy volunteers were selected for the study and each volunteer was administered either the test or reference formulation with 240 ml of water after a high fat standard breakfast. Blood samples were taken at regular intervals for a period of 24 hours. A cross over study was done after a period of one week.

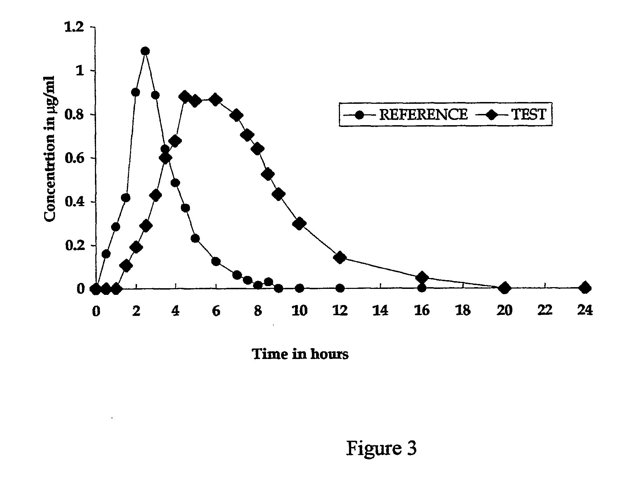

Figure 3 shows a plot of blood level concentration of zidovudine for the reference product (Reference) in comparison with a zidovidudine modified release composition according to the present invention (Test).

The present invention is illustrated by the following examples.

### Examples

Lamivudine, zidovudine, hydrophilic polymers, calcium sulphate, dicalcium phosphate/polyvinyl pyrrolidone, and microcrystalline cellulose were screened through a 30 mesh sieve and mixed with magnesium stearate. The blend was compacted and the slugs obtained were milled to form granules. The sized granules were blended with the fines and the remaining lubricant and further compressed into tablets.

The tablets were tested for lamivudine and zidovudine release in 900 ml of 0.1 N hydrochloric acid for 2 hrs, after which the dissolution medium was changed to pH 6.8 phosphate buffer 900 ml maintained at 37±1°C. The tablets were placed into a 40 mesh basket (USP apparatus type-I) and were rotated at 100 rpm. At each sampling point aliquots of dissolution medium were withdrawn and replaced with fresh medium at each interval.

### Example 1

| **Ingredients** | **Weight (mg/tablet)** | **% w/w** |
|---|---|---|
| Lamivudine | 300.00 | 24.0 |
| Zidovudine | 600.00 | 48.0 |
| Microcrystalline cellulose | 200.00 | 16.0 |
| Hydroxypropyl methylcellulose (Methocel K15 MP) | 50.00 | 4.0 |
| Sodium alginate (Keltone HVCR) | 31.25 | 2.5 |
| Guar gum (Meypro-Guar CSAA M-175) | 6.25 | 0.5 |
| Calcium sulphate | 3.75 | 0.3 |
| Dicalcium phosphate | 40.00 | 3.2 |
| Magnesium stearate | 18.75 | 1.5 |

| **Time (hour)** | **% actives released** | |
|---|---|---|
| | **Lamivudine** | **Zidovudine** |
| 1 | 37.0 | 30.6 |
| 2 | 50.1 | 38.6 |
| 3 | 63.5 | 46.8 |
| 4 | 73.2 | 53.5 |
| 6 | 87.7 | 65.3 |
| 8 | 97.6 | 75.3 |
| 10 | 103.7 | 85.8 |

### Example 2

| **Ingredients** | **Weight (mg/tablet)** | **% w/w** |
|---|---|---|
| Lamivudine | 300.00 | 24.00 |
| Zidovudine | 600.00 | 48.00 |
| Microcrystalline cellulose | 173.75 | 13.90 |
| Hydroxypropyl methylcellulose (Methocel K15 MP) | 68.75 | 5.50 |
| Sodium alginate (Keltone HVCR) | 37.50 | 3.00 |
| Guar gum (Meypro-Guar CSAA M-175) | 12.50 | 1.00 |
| Calcium sulphate | 4.50 | 0.36 |
| Dicalcium phosphate | 40.00 | 3.20 |
| Magnesium stearate | 13.00 | 1.04 |

| **Time (hour)** | **% actives released** | |
|---|---|---|
| | **Lamivudine** | **Zidovudine** |
| 1 | 34.0 | 23.1 |
| 2 | 47.4 | 30.6 |
| 3 | 57.1 | 36.7 |
| 4 | 64.6 | 41.7 |
| 6 | 79.3 | 52.8 |
| 8 | 89.6 | 62.5 |
| 10 | 97.0 | 72.5 |
| 12 | 102.3 | 82.1 |
| 14 | 106.3 | 90.9 |

### Example 3

| **Ingredients** | **Weight (mg/tablet)** | **% w/w** |
|---|---|---|
| Lamivudine | 300.0 | 24.00 |
| Zidovudine | 600.0 | 48.00 |
| Microcrystalline cellulose | 167.5 | 13.40 |
| Hydroxypropyl methylcellulose (Methocel K15 MP) | 50.0 | 4.00 |
| Sodium alginate (Keltone HVCR) | 50.0 | 4.00 |
| Guar gum (Meypro-Guar CSAA M-175) | 25.0 | 2.00 |
| Calcium sulphate | 4.5 | 0.36 |
| Dicalcium phosphate | 40.0 | 3.20 |
| Magnesium stearate | 13.0 | 1.04 |

| **Time (hour)** | **% actives released** | |
|---|---|---|
| | **Lamivudine** | **Zidovudine** |
| 1 | 32.3 | 22.6 |
| 2 | 45.5 | 29.0 |
| 3 | 54.4 | 34.4 |
| 4 | 64.1 | 39.9 |
| 6 | 79.4 | 50.5 |
| 8 | 94.3 | 62.0 |
| 10 | 102.6 | 70.6 |
| 12 | | 78.2 |
| 14 | | 85.6 |
| 16 | | 91.7 |

### Example 4

| **Ingredients** | **Weight (mg/tablet)** | **% w/w** |
|---|---|---|
| Lamivudine | 300.0 | 24.00 |
| Zidovudine | 600.0 | 48.00 |
| Microcrystalline cellulose | 155.0 | 12.40 |
| Hydroxypropyl methylcellulose (Methocel K15 MP) | 50.0 | 4.00 |
| Sodium alginate (Keltone HVCR) | 37.5 | 3.00 |
| Guar gum (Meypro-Guar CSAA M-175) | 50.0 | 4.00 |
| Calcium sulphate | 4.5 | 0.36 |
| Dicalcium phosphate | 40.0 | 3.20 |
| Magnesium stearate | 13.0 | 1.04 |

| **Time (hour)** | **% actives released** | |
|---|---|---|
| | **Lamivudine** | **Zidovudine** |
| 1 | 32.0 | 25.3 |
| 2 | 48.2 | 33.8 |
| 3 | 57.1 | 39.6 |
| 4 | 64.8 | 43.6 |
| 6 | 72.4 | 52.0 |
| 8 | 87.9 | 59.4 |
| 10 | 95.5 | 67.1 |
| 12 | 101.6 | 75.2 |
| 14 | | 81.9 |

### Example 5

| **Ingredients** | **Weight (mg/tablet)** | **% w/w** |
|---|---|---|
| Lamivudine | 300.0 | 24.00 |
| Zidovudine | 600.0 | 48.00 |
| Microcrystalline cellulose | 170.0 | 13.60 |
| Hydroxypropyl methylcellulose (Methocel K15 MP) | 75.0 | 6.00 |
| Sodium alginate (Keltone HVCR) | 37.5 | 3.00 |
| Guar gum (Meypro-Guar CSAA M-175) | 25.0 | 2.00 |
| Calcium sulphate | 4.50 | 0.36 |
| Polyvinyl Pyrrolidone | 25.0 | 2.00 |
| Magnesium stearate | 13.0 | 1.04 |

| **Time (hour)** | **% actives released** | |
|---|---|---|
| | **Lamivudine** | **Zidovudine** |
| 1 | 24.0 | 12.3 |
| 2 | 36.8 | 19.4 |
| 3 | 46.3 | 25.2 |
| 4 | 53.6 | 29.9 |
| 6 | 67.4 | 39.6 |
| 8 | 78.3 | 48.5 |
| 10 | 85.2 | 55.6 |
| 12 | 91.2 | 63.0 |
| 14 | 97.7 | 71.8 |
| 16 | | 73.8 |

### Example 6

| **Ingredients** | **Weight (mg/tablet)** | **% w/w** |
|---|---|---|
| Lamivudine | 300.00 | 24.00 |
| Zidovudine | 600.00 | 48.00 |
| Microcrystalline cellulose | 130.00 | 10.40 |
| Hydroxypropyl methylcellulose (Methocel K15 MP) | 75.00 | 6.00 |
| Sodium alginate (Keltone HVCR) | 37.50 | 3.00 |
| Guar gum (Meypro-Guar CSAA M-175) | 50.00 | 4.00 |
| Calcium sulphate | 4.50 | 0.36 |
| Dicalcium phosphate | 40.00 | 3.20 |
| Magnesium stearate | 13.00 | 1.04 |

| **Time (hour)** | **% actives released** | |
|---|---|---|
| | **Lamivudine** | **Zidovudine** |
| 1 | 35.3 | 24.9 |
| 2 | 47.3 | 32.1 |
| 3 | 54.6 | 36.3 |
| 4 | 64.5 | 42.3 |
| 6 | 77.5 | 51.6 |
| 8 | 87.3 | 59.8 |
| 10 | 93.8 | 66.8 |
| 12 | 99.2 | 74.5 |
| 14 | 99.5 | 79.6 |

### Example 7

| **Ingredients** | **Weight (mg/tablet)** | **% w/w** |
|---|---|---|
| Lamivudine | 300.0 | 24.00 |
| Zidovudine | 600.0 | 48.00 |
| Microcrystalline cellulose | 155.0 | 12.40 |
| Hydroxypropyl methylcellulose (Methocel K15 MP) | 75.0 | 6.00 |
| Sodium alginate (Keltone HVCR) | 37.5 | 3.00 |
| Guar gum (Meypro-Guar CSAA M-175) | 25.0 | 2.00 |
| Calcium sulphate | 4.50 | 0.36 |
| Dicalcium phosphate | 40.0 | 3.20 |
| Magnesium stearate | 13.0 | 1.04 |

| **Time (hour)** | **% actives released** | |
|---|---|---|
| | **Lamivudine** | **Zidovudine** |
| 1 | 33.2 | 20.0 |
| 2 | 45.8 | 26.5 |
| 3 | 56.2 | 32.7 |
| 4 | 67.2 | 39.4 |
| 6 | 82.7 | 50.3 |
| 8 | 94.9 | 62.0 |
| 10 | 105.1 | 72.4 |
| 12 | | 81.7 |
| 14 | | 91.4 |

### Example 8

| **Ingredients** | **Weight (mg/tablet)** | **% w/w** |
|---|---|---|
| Lamivudine | 300.00 | 24.00 |
| Zidovudine | 600.00 | 48.00 |
| Microcrystalline cellulose | 214.75 | 17.18 |
| Hydroxypropyl methylcellulose (Methocel K15 MP) | 50.00 | 4.00 |
| Sodium alginate (Keltone HVCR) | 18.75 | 1.50 |
| Guar gum (Meypro-Guar CSAA M-175) | 6.25 | 0.50 |
| Calcium sulphate | 2.25 | 0.18 |
| Dicalcium phosphate | 40.00 | 3.20 |
| Magnesium stearate | 18.00 | 1.44 |

| **Time (hour)** | **% actives released** | |
|---|---|---|
| | **Lamivudine** | **Zidovudine** |
| 1 | 58.0 | 53.6 |
| 2 | 72.3 | 64.5 |
| 3 | 81.3 | 71.4 |
| 4 | 88.3 | 77.8 |
| 6 | 97.3 | 88.0 |
| 8 | 104.3 | 97.2 |

### Example 9

| **Ingredients** | **Weight (mg/tablet)** | **% w/w** |
|---|---|---|
| Lamivudine | 300.00 | 46.15 |
| Microcrystalline cellulose | 187.00 | 28.77 |
| Hydroxypropyl methylcellulose (Methocel K15 MP) | 62.50 | 9.62 |
| Sodium alginate (Keltone HVCR) | 31.25 | 4.81 |
| Guar gum (Meypro-Guar CSAA M-175) | 12.50 | 1.92 |
| Calcium sulphate | 3.75 | 0.58 |
| Dicalcium phosphate | 46.50 | 7.15 |
| Magnesium stearate | 6.50 | 1.00 |

| **Time (hour)** | **% Lamivudine released** |
|---|---|
| 2 | 45.6 |
| 4 | 68.2 |
| 6 | 77.4 |
| 8 | 92.8 |
| 10 | 98.8 |
| 12 | 102.6 |

### Example 10

| **Ingredients** | **Weight (mg/tablet)** | **% w/w** |
|---|---|---|
| Zidovudine | 600.00 | 63.16 |
| Microcrystalline cellulose | 187.00 | 19.68 |
| Hydroxypropyl methylcellulose (Methocel K15 MP) | 62.50 | 6.58 |
| Sodium alginate (Keltone HVCR) | 31.25 | 3.29 |
| Guar gum (Meypro-Guar CSAA M-175) | 12.50 | 1.32 |
| Calcium sulphate | 3.75 | 0.39 |
| Dicalcium phosphate | 43.50 | 4.58 |
| Magnesium stearate | 9.50 | 1.00 |

| **Time (hour)** | **% Zidovudine released** |
|---|---|
| 2 | 36.6 |
| 4 | 55.0 |
| 6 | 63.1 |
| 8 | 71.6 |
| 10 | 77.9 |
| 12 | 83.6 |
| 14 | 89.8 |

## Claims

1. A pharmaceutical composition for controlled release of active ingredient comprising:
i. 4% to 50% by wt. of lamivudine or a pharmaceutically acceptable derivative thereof as the first active ingredient;
ii. 8% to 80% by wt. of zidovudine or a pharmaceutically acceptable derivative thereof as the second active ingredient;
iii. a controlled release matrix comprising a mixture of hydrophilic polymers consisting of 2% to 12% of at least one hydroxypropyl methylcellulose, 0.5% to 10% by wt. of at least one sodium alginate and 0.1 to 10% by wt. of at least one guar gum; and
iv. a pharmaceutically acceptable calcium salt.

2. A composition as claimed in claim 1, wherein the composition comprises from 4% to 50% by weight of lamivudine or a pharmaceutically acceptable derivative thereof, 8% to 80% by weight of zidovudine or a pharmaceutically acceptable derivative thereof, 1 % to 20% by weight of said mixture of hydrophilic polymers comprising sodium alginate in an amount from 0.5% to 10% by weight, guar gum in an amount from 0.1 % to 10% by weight, hydroxypropyl methylcellulose in an amount from 2% to 12% by weight and calcium sulphate in an amount from 0.1% to 1.8 % by weight.

3. A composition as claimed in claim 1, wherein the composition comprises from 4% to 50% by weight of lamivudine or a pharmaceutically acceptable derivative thereof, 8% to 80% by weight of zidovudine or a pharmaceutically acceptable derivative thereof, 1 % to 15% by weight of said mixture of hydrophilic polymers comprising of sodium alginate in an amount from 1% to 6% by weight, guar gum in an amount from 0.5% to 6% by weight, hydroxypropyl methylcellulose in an amount from 3% to 8% by weight and calcium sulphate in an amount from 0.1 % to 1.2% by weight.

4. A composition as claimed in claim 1, 2 or 3 further comprising at least one water dispersible or water-soluble diluent.

5. A composition as claimed in claim 4 wherein the diluent is present in an amount of from 1 % to 28% by weight.

6. A composition as claimed in claim 4 or 5 wherein the diluent is microcrystalline cellulose in an amount from 5% to 20% by weight.

7. A composition as claimed in claim 4 or 5 wherein the diluent is dicalcium phosphate in an amount from 1% to 5% by weight.

8. A pharmaceutical composition for controlled release of an active ingredient, said composition comprising:
i. an active ingredient selected from amongst lamivudine, zidovudine and combinations of lamivudine and zidovudine or their derivatives;
ii. a controlled release matrix comprising a mixture of hydrophilic polymers consisting of 2% to 15% by weight of at least one hydroxypropyl methylcellulose, 0.5 to 12% by wt. of at least one sodium alginate and 0.1 to 10% by wt. of at least one guar gum;
iii. a pharmaceutically acceptable calcium salt.

9. A composition as claimed in claim 8, wherein the pharmaceutically acceptable active ingredient is lamivudine or a pharmaceutically acceptable derivative thereof.

10. A composition as claimed in claim 8, wherein the pharmaceutically acceptable active ingredient is zidovudine or a pharmaceutically derivative thereof.

11. A composition as claimed in claim 1 or claim 8 wherein the amount of lamivudine or pharmaceutically acceptable derivative thereof is from 50 mg to 500 mg, preferably 300 mg.

12. A composition as claimed in claim 1 or claim 9 wherein the amount of zidovudine or pharmaceutically acceptable derivative thereof is from 100 mg to 1000 mg, preferably 600 mg.

13. A composition as claimed in Claim 1 or Claim 8, wherein the pharmaceutically acceptable calcium salt is selected from the group consisting of calcium sulphate, calcium phosphate, calcium carbonate and calcium chloride; preferably calcium sulphate.

14. A composition as claimed in any of claims 8 to 10 or claims 11 to 13 when dependent on claim 8 further comprising at least one water dispersible or water-soluble diluent.

15. A composition as claimed in claim 14 wherein the diluent is present in an amount of from 1 % to 55% by weight.

16. A composition as claimed in claim 14 or 15 wherein the diluent is microcrystalline cellulose in an amount from 5% to 30% by weight.

17. A composition as claimed in claim 14 or 15 wherein the diluent is dicalcium phosphate in an amount from 1 % to 10% by weight.

18. A pharmaceutical composition for controlled release of an active ingredient, said composition comprises an active ingredient, selected from amongst lamivudine, zidovudine or their pharmaceutically acceptable derivatives, and a control release matrix comprising a mixture of hydrophilic polymers consisting of hydroxypropyl methylcellulose, sodium alginate and guar gum and a pharmaceutically acceptable calcium salt wherein said composition comprises 4% to 80% by weight of said active ingredient, and 1% to 25% by weight of said mixture of hydrophilic polymers, wherein said mixture of hydrophilic polymers consists of 0.5% to 12% by weight of sodium alginate, 0.1% to 10% by weight of guar gum, 2% to 15% by weight of hydroxypropyl methylcellulose and 0.1% to 1.8% by weight of said pharmaceutically acceptable calcium salt.

19. A composition as claimed in claim 18, wherein the composition comprises from 4% to 80% by weight of said active ingredient, and 1% to 20% by weight of said mixture of hydrophilic polymers consisting of sodium alginate in an amount from 2% to 8% by weight, guar gum in an amount from 1% to 4% by weight, hydroxypropyl methylcellulose in an amount from 5% to 10% by weight and calcium sulphate in an amount from 0.1 % to 1.2% by weight.

20. A composition as claimed in claim 18 or 19 further comprising at least one water dispersible or water-soluble diluent.

21. A pharmaceutical composition as claimed in claim 8, including lamivudine, zidovudine or combinations thereof, wherein the time taken to reach the peak plasma concentration (T max) of the said actives either alone or in combination is in the range of from 3 to 7 hours.

22. An extended release pharmaceutical composition as claimed in claim 8 comprising lamivudine with or without zidovudine wherein the rate of release of lamivudine from the dosage form at 37°C in USP apparatus 1 at 100 rpm in 0.1 N HCl for 2 hours followed by phosphate buffer (pH 6.8) is:
| Time (hrs) | % drug release |
|---|---|
| 2 | 25-55 |
| 4 | 35 - 75 |
| 8 | 45-95 |
| 10 | NLT 70% |
| 12 | NLT 80% |

23. An extended release pharmaceutical composition as claimed in claim 8 comprising zidovudine, with or without lamivudine, wherein the rate of release of zidovudine from the dosage from at 37°C in USP apparatus I, at 100 rpm in 0.1 HCl for 2 hours followed by phosphate buffer (pH 6.8) is:
| Time (hrs) | % drug release |
|---|---|
| 2 | 15 - 45 |
| 4 | 25 - 60 |
| 8 | 45 - 80 |
| 12 | 60-95 |
| 14 | NLT 75 |

24. A process for the preparation of a pharmaceutical composition comprising mixing together active ingredients selected from amongst lamivudine, zidovudine or mixtures thereof with a controlled release matrix comprising of mixture of hydrophilic polymers consisting of 2% to 15% of at least one hydroxypropyl methylcellulose, 0.5 to 12% by wt. of at least one sodium alginate, 0.1 to 10% by wt. of at least one guar gum or mixtures thereof, and with a pharmaceutically acceptable calcium salt, optionally a diluent and a lubricant, to form a blend, and compressing the resulting blend into tablets.

25. A process as claimed in claim 24, wherein the blend is dry granulated prior to compression.

26. A process as claimed in claim 24, wherein the blend is wet granulated prior to compression.

27. A composition as claimed in any preceding claim in the form of a tablet.

## Patentansprüche

1. Pharmazeutische Zusammensetzung zur kontrollierten Freisetzung von Wirkstoff enthaltend:
i. 4 Gew.-% bis 50 Gew.-% Lamivudin oder ein pharmazeutisch annehmbares Derivat davon als den ersten Wirkstoff;
ii. 8 Gew.-% bis 80 Gew.-% Zidovudin oder ein pharmazeutisch annehmbares Derivat davon als den zweiten Wirkstoff;
iii. eine kontrolliert freisetzende Matrix, enthaltend eine Mischung aus hydrophilen Polymeren, bestehend aus 2 % bis 12 % wenigstens einer Methylcellulose, 0,5 Gew.-% bis 10 Gew.-% wenigstens eines Natriumalginats und 0,1 Gew.-% bis 10 Gew.-% wenigstens einem Guargummi; und
iv. ein pharmazeutisch annehmbares Calciumsalz.

2. Zusammensetzung, wie in Anspruch 1 beansprucht, worin die Zusammensetzung zwischen 4 Gew.-% bis 50 Gew.-% Lamivudin oder ein pharmazeutisch annehmbares Derivat davon, 8 Gew.-% bis 80 Gew.-% Zidovudin oder ein pharmazeutisch annehmbares Derivat davon, 1 Gew.-% bis 20 Gew.-% der Mischung von hydrophilen Polymeren, bestehend aus Natriumalginat in einer Menge von 0,5 Gew.-% bis 10 Gew.-%, Guargummi in einer Menge von 0,1 Gew.-% bis 10 Gew.-%, Hydroxypropylmethylcellulose in einer Menge von 2 Gew.-% bis 12 Gew.-% und Calciumsulfat in einer Menge von 0,1 Gew.-% bis 1,8 Gew.-% enthält.

3. Zusammensetzung, wie in Anspruch 1 beansprucht, worin die Zusammensetzung zwischen 4 Gew.-% bis 50 Gew.-% Lamivudin oder ein pharmazeutisch annehmbares Derivat davon, 8 Gew.-% bis 80 Gew.-% Zidovudin oder ein pharmazeutisch annehmbares Derivat davon, 1 Gew.-% bis 15 Gew.-% der Mischung von hydrophilen Polymeren, bestehend aus Natriumalginat in einer Menge von 1 Gew.-% bis 6 Gew.-%, Guargummi in einer Menge von 0,5 Gew.-% bis 6 Gew.-%, Hydroxypropylmethylcellulose in einer Menge von 3 Gew.-% bis 8 Gew.-% und Calciumsulfat in einer Menge von 0,1 Gew.-% bis 1,2 Gew.-% enthält.

4. Zusammensetzung, wie in Anspruch 1, 2 oder 3 beansprucht, weiterhin enthaltend wenigstens ein in Wasser dispergierbares oder wasserlösliches Streckmittel.

5. Zusammensetzung, wie in Anspruch 4 beansprucht, worin das Streckmittel in einer Menge von 1 Gew.-% bis 28 Gew.-% vorliegt.

6. Zusammensetzung, wie in Anspruch 4 oder 5 beansprucht, worin das Streckmittel Mikrokristalline Cellulose in einer Menge von 5 Gew.-% bis 20 Gew.-% ist.

7. Zusammensetzung, wie in Anspruch 4 oder 5 beansprucht, worin das Streckmittel Dicalciumphosphat in einer Menge von 1 Gew.-% bis 5 Gew.-% ist.

8. Pharmazeutische Zusammensetzung zur kontrollierten Freisetzung eines Wirkstoffs enthaltend:
i. einen Wirkstoff ausgewählt aus Lamivudin, Zidovudin und Kombinationen aus Lamivudin und Zidovudin oder deren Derivate;
ii. eine kontrolliert freisetzende Matrix, enthaltend eine Mischung aus hydrophilen Polymeren, bestehend aus 2 % bis 12 % wenigstens einer Hydroxypropylmethylcellulose, 0,5 Gew.-% bis 12 Gew.-% wenigstens eines Natriumalginats und 0,1 Gew.-% bis 10 Gew.-% wenigstens einem Guargummi;
iii. ein pharmazeutisch annehmbares Calciumsalz.

9. Zusammensetzung, wie in Anspruch 8 beansprucht, worin der pharmazeutisch annehmbare Wirkstoff Lamivudin oder ein pharmazeutisch annehmbares Derivat davon ist.

10. Zusammensetzung, wie in Anspruch 8 beansprucht, worin der pharmazeutisch annehmbare Wirkstoff Zidovudin oder ein pharmazeutisch annehmbares Derivat davon ist.

11. Zusammensetzung, wie in Anspruch 1 oder in Anspruch 8 beansprucht, worin die Menge an Lamivudin oder dessen pharmazeutisch annehmbaren Derivats zwischen 50 mg bis 500 mg, vorzugsweise 300 mg, ist.

12. Zusammensetzung, wie in Anspruch 1 oder in Anspruch 9 beansprucht, worin die Menge an Zidovudin oder dessen pharmazeutisch annehmbaren Derivats zwischen 100 mg bis 1000 mg, vorzugsweise 600 mg, ist.

13. Zusammensetzung, wie in Anspruch 1 oder in Anspruch 8 beansprucht, worin das pharmazeutisch annehmbare Calciumsalz aus der Gruppe ausgewählt ist, bestehend aus Calciumsulfat, Calciumphosphat, Calciumcarbonat und Calciumchlorid; vorzugsweise Calciumsulfat ist.

14. Zusammensetzung, wie in irgendeinem der Ansprüche 8 bis 10 oder der Ansprüche 11 bis 13 beansprucht, wenn abhängig von Anspruch 8, weiterhin enthaltend wenigstens ein in Wasser dispergierbares oder wasserlösliches Streckmittel.

15. Zusammensetzung, wie in Anspruch 14 beansprucht, worin das Streckmittel in einer Menge von 1 Gew.-% bis 55 Gew.-% vorliegt.

16. Zusammensetzung, wie in Anspruch 14 oder 15 beansprucht, worin das Streckmittel Mikrokristalline Cellulose in einer Menge von 5 Gew.-% bis 30 Gew.-% ist.

17. Zusammensetzung, wie in Anspruch 14 oder 15 beansprucht, worin das Streckmittel Dicalciumphosphat in einer Menge von 1 Gew.-% bis 10 Gew.-% ist.

18. Pharmazeutische Zusammensetzung zur kontrollierten Freisetzung eines Wirkstoffes, enthaltend einen Wirkstoff, ausgewählt aus Lamivudin, Zidovudin oder deren pharmazeutisch annehmbaren Derivaten, und einer kontrolliert freisetzenden Matrix, enthaltend eine Mischung von hydrophilen Polymeren bestehend aus Hydroxypropylmethylcellulose, Natriumalginat und Guargummi und einem pharmazeutisch annehmbarem Calciumsalz, worin die Zusammensetzung 4 Gew.-% bis 80 Gew.-% des Wirkstoffs, und 1 Gew.-% bis 25 Gew.-% der Mischung der hydrophilen Polymere, worin die Mischung der hydrophilen Polymere aus 0,5 Gew.-% bis 12 Gew.-% Natriumalginat, 0,1 Gew.-% bis 10 Gew.-% Guargummi, 2 Gew.-% bis 15 Gew.-% Hydroxypropylmethylcellulose besteht, und 0,1 Gew.-% bis 1,8 Gew.-% des pharmazeutisch annehmbarem Calciumsalzes enthält.

19. Zusammensetzung, wie in Anspruch 18 beansprucht, worin die Zusammensetzung 4 Gew.-% bis 80 Gew.-% des Wirkstoffs, und 1 Gew.-% bis 20 Gew.-% der Mischung der hydrophilen Polymere, worin die Mischung der hydrophilen Polymere aus Natriumalginat in einer Menge von 2 Gew.-% bis 8 Gew.-%, Guargummi in einer Menge von 1 Gew.-% bis 4 Gew.-%, Hydroxypropylmethylcellulose in einer Menge von 5 Gew.-% bis 10 Gew.-% besteht, und pharmazeutisch annehmbares Calciumsalz in einer Menge von 0,1 Gew.-% bis 1,2 Gew.-% enthält.

20. Zusammensetzung, wie in Anspruch 18 oder 19 beansprucht, weiterhin enthaltend wenigstens ein in Wasser dispergierbares oder wasserlösliches Streckmittel.

21. Pharmazeutische Zusammensetzung, wie in Anspruch 8 beansprucht, enthaltend Lamivudin, Zidovudin oder deren Kombinationen, worin die erforderliche Zeit, um die Peak-Plasma-Konzentration (T max) der Wirkstoffe, allein oder in Kombination, zu erreichen, im Bereich von 3 bis 7 Stunden liegt.

22. Verlängert freisetzende pharmazeutische Zusammensetzung, wie in Anspruch 8 beansprucht, umfassend Lamivudin, mit oder ohne Zidovudin, worin die Freisetzungsrate von Lamivudin aus der Dosierungsform bei 37 °C im USP-Apparat 1 bei 100 upm in 0,1 N HCl für 2 Stunden, gefolgt von Phosphatpuffer (pH 6,8)ist:
| Zeit (Stunden) | % Arzneimittelfreisetzung |
|---|---|
| 2 | 25 -55 |
| 4 | 35 - 75 |
| 8 | 45 - 95 |
| 10 | nicht weniger als 70% |
| 12 | nicht weniger als 80% |

23. Verlängert freisetzende pharmazeutische Zusammensetzung, wie in Anspruch 8 beansprucht, umfassend Zidovudin, mit oder ohne Lamivudin, worin die Freisetzungsrate von Zidovudin aus der Dosierungsform bei 37 °C im USP-Apparat 1 bei 100 upm in 0,1 N HCl für 2 Stunden, gefolgt von Phosphatpuffer (pH 6,8)ist:
| Zeit (Stunden) | % Arzneimittelfreisetzung |
|---|---|
| 2 | 15 -45 |
| 4 | 25 - 60 |
| 8 | 45 - 80 |
| 12 | 60 - 95 |
| 14 | nicht weniger als 75% |

24. Verfahren zur Herstellung einer pharmazeutischen Zusammensetzung umfassend das Mischen von Wirkstoffen ausgewählt unter Lamivudin, Zidovudin oder deren Mischungen, mit einer kontrolliert freisetzenden Matrix, enthaltend eine Mischung aus hydrophilen Polymeren, bestehend aus 2 % bis 15 % wenigstens einer Hydroxypropylmethylcellulose, 0,5 Gew.-% bis 12 Gew.-% wenigstens eines Natriumalginats, 0,1 Gew.-% bis 10 Gew.-% wenigstens eines Guargummis oder deren Mischungen, und mit einem pharmazeutisch annehmbaren Calciumsalz, optional einem Streckmittel und einem Gleitmittel, um eine Mischung zu bilden, und Pressen der erhaltenen Mischung zu Tabletten.

25. Verfahren, wie in Anspruch 24 beansprucht, worin die Mischung vor dem Pressen trocken granuliert wird.

26. Verfahren, wie in Anspruch 24 beansprucht, worin die Mischung vor dem Pressen feucht granuliert wird.

27. Zusammensetzung, wie in irgendeinem der voranstehenden Ansprüche beansprucht, in Form einer Tablette.

## Revendications

1. Composition pharmaceutique pour la diffusion contrôlée d'un ingrédient actif comprenant :
i. 4 à 5% en poids de lamivudine ou un dérivé de celui-ci acceptable sur le plan pharmaceutique en tant que premier ingrédient actif ;
ii. 8 à 80% en poids de zidovudine ou d'un dérivé acceptable sur le plan pharmaceutique de celui-ci, en tant que second ingrédient actif ;
iii. une matrice de diffusion contrôlée comprenant un mélange de polymères hydrophiles consistant en 2 à 12% d'au moins une méthylcellulose hydroxy propyle, 0,5% à 10% en poids d'au moins un alginate sodium et 0,1 à 10% en poids d'au moins une gomme guar ; et
iv. un sel de calcium acceptable sur le plan pharmaceutique.

2. Composition selon la revendication 1, **caractérisée en ce que** la composition comporte 4 à 50% en poids de lamivudine ou d'un dérivé pharmaceutiquement acceptable de celui-ci, 8 à 80% de zidovudine ou un dérivé acceptable pharmaceutiquement de celui-ci, 1 à 20% en poids dudit mélange de polymères d'hydrophiles comprenant un alginate de sodium dans une quantité de 0,5 à 10% en poids, de la gomme guar dans une quantité de 0,1 à 10% en poids, de la méthylcellulose hydroxy propyle dans une quantité de 2 à 12% en poids et du sulfate de calcium en quantité de 0,1 à 1,8% en poids.

3. Composition selon la revendication 1, **caractérisée en ce qu'**elle comporte 4 à 50% en poids de lamivudine ou d'un dérivé pharmaceutiquement acceptable de celui-ci, 8 à 80% en poids de zidovudine ou d'un dérivé acceptable pharmaceutiquement de celui-ci, 1 à 15% en poids dudit mélange de polymères hydrophiles comprenant un alginate de sodium dans une quantité de 1 à 6% en poids, de la gomme guar dans une quantité de 0,5 à 6% en poids, de la méthylcellulose hydroxy propyle dans une quantité de 3 à 8% en poids et du sulfate de calcium en quantité de 0,1 à 1,2% en poids.

4. Composition selon la revendication 1, 2 ou 3, **caractérisée en ce qu'**elle comporte en outre au moins un diluant dispersible dans l'eau ou soluble dans l'eau.

5. Composition selon la revendication 4, **caractérisée en ce que** le diluant est présent dans une quantité de 1 à 28% en poids.

6. Composition selon la revendication 4 ou 5, **caractérisée en ce que** le diluant est de la cellulose microcristalline dans une quantité de 5 à 20% en poids.

7. Composition selon la revendication 4 ou 5, **caractérisée en ce que** le diluant est du phosphate dicalcium dans une quantité de 1 à 5 % en poids.

8. Composition pharmaceutique pour la diffusion contrôlée d'un ingrédient actif, ladite composition comprenant :
i. un ingrédient actif choisi parmi lamivudine, zidovudine et les combinaisons de lamivudine et zidovudine ou autres dérivés.
ii. une matrice de diffusion contrôlée comprenant un mélange de polymères hydrophiles consistant en 2 à 15% en poids d'au moins un méthylcellulose hydroxy propyle, 0,5 à 12% en poids d'au moins un alginate de sodium et 0,1 à 10% en poids d'au moins une gomme guar.
iii. un sel de calcium acceptable sur le plan pharmaceutique.

9. Composition selon la revendication 8, **caractérisée en ce que** l'ingrédient actif acceptable pharmaceutiquement est lamivudine ou un dérivé de celui-ci acceptable sur le plan pharmaceutique.

10. Composition selon la revendication 8, **caractérisée en ce que** l'ingrédient actif acceptable pharmaceutiquement est zidovudine ou un dérivé acceptable sur le plan pharmaceutique de celui-ci.

11. Composition selon la revendication 1 ou 8, **caractérisée en ce que** la quantité de lamivudine ou du dérivé acceptable pharmaceutiquement de celle-ci est de 50 mg à 500 mg, de préférence 300 mg.

12. Composition selon la revendication 1 ou 9, **caractérisée en ce que** la quantité de zidovudine ou du dérivé acceptable pharmaceutiquement de celui-ci est de 100 mg à 1000 mg, de préférence 600 mg.

13. Composition selon la revendication 1 ou 8, **caractérisée en ce que** le sel de calcium acceptable pharmaceutiquement est choisi parmi le groupe consistant en sulfate de calcium, phosphate de calcium, carbonate de calcium, et chlorure de calcium, de préférence sulfate de calcium.

14. Composition selon l'une des revendications 8 à 10 ou les revendications 11 à 13 lorsqu'elle est dépendante de la revendication 8, **caractérisée en ce qu'**elle comporte en outre au moins un diluant dispersible dans l'eau ou soluble dans l'eau.

15. Composition selon la revendication 14, **caractérisée en ce que** le diluant est présent dans une quantité de 1 à 55% en poids.

16. Composition selon la revendication 14 ou 15, **caractérisée en ce que** le diluant est de la cellulose microcristalline dans une quantité de 5 à 30% en poids.

17. Composition selon la revendication 14 ou 15, **caractérisée en ce que** le diluant est du phosphate dicalcium dans une quantité de 1 à 10 % en poids.

18. Composition pharmaceutique pour la diffusion contrôlée d'un ingrédient actif, ladite composition comprenant un ingrédient actif, choisi parmi le groupe incluant lamivudine, zidovudine ou autres dérivés pharmaceutiquement acceptables, et une matrice de diffusion contrôlée comprenant un mélange de polymères hydrophiles consistant en méthylcellulose hydroxy propyle, alginate de sodium et gomme guar et un sel de calcium acceptable pharmaceutiquement, **caractérisée en ce que** ladite composition comporte 4 à 8 % en poids dudit ingrédient actif, entre 1 et 25% en poids dudit mélange de polymères hydrophiles, et **en ce que** ledit mélange de polymères hydrophiles consiste en 0,5 à 12% en poids de alginate sodium, 0,1 à 10% en poids de gomme guar, 2 à 15% en poids de méthylcellulose hydroxy propyle et 0,1% à 1,8% en poids dudit sel de calcium acceptable sur le plan pharmaceutique.

19. Composition selon la revendication 18, **caractérisée en ce que** la composition comporte 4 à 80% en poids dudit ingrédient actif, et 1 à 20% en poids dudit mélange de polymères hydrophiles consistant d'alginate de sodium dans une quantité de 2 à 8% en poids, gomme guar dans une quantité de 1 à 4% en poids, méthylcellulose hydroxy propyle dans une quantité de 5 à 10% en poids et sulfate de calcium dans une quantité de 0,1 à 1,2% en poids.

20. Composition selon la revendication 18 ou 19, **caractérisée en ce que** la composition comporte en outre au moins un diluant dispersible dans l'eau ou soluble dans l'eau.

21. Composition pharmaceutique selon la revendication 8, incluant lamivudine, zidovudine ou les combinaisons de celui-ci, **caractérisée en ce que** le temps pris pour atteindre le pic de concentration plasma (T max) desdits actifs, soit en combinaison ou seul, est dans la gamme de 3 à 7 heures.

22. Composition pharmaceutiquement étendue pour le relâchement, selon la revendication 8, comprenant lamivudine avec ou sans zidovudine, **caractérisée en ce que** le taux de relâchement de lamivudine à partir du dosage de forme à 37%, dans un appareil USP de 1 à 100 tours par minute dans 0,1 N HCl pendant 2 heures suivi par un tampon de phosphate (pH 6,8) est :
| Temps (heures) | % de relâchement |
|---|---|
| 2 | 25-55 |
| 4 | 35-75 |
| 8 | 45-95 |
| 10 | NLT 70% |
| 12 | NLT 80% |

23. Composition pharmaceutique pour la diffusion étendue selon la revendication 8, et comprenant zidovudine, avec ou sans lamivudine, tandis que le taux de diffusion de zidovudine à partir du dosage à partir de 37°C dans un appareil USP I, à 100 tours par minute dans 0,1 HCl pendant 2 heures suivi par un tamponnage phosphate (pH 6,8) est :
| Temps (heures) | % de relâchement |
|---|---|
| 2 | 15-45 |
| 4 | 25-60 |
| 8 | 45-80 |
| 12 | 60-95 |
| 14 | NLT 75 |

24. Procédé pour la préparation d'une composition pharmaceutique comprenant le mélange ensemble d'ingrédients actifs choisis parmi lamivudine, zidovudine, ou mélange de celui-ci avec une matrice à diffusion contrôlée comprenant un mélange de polymères hydrophiles consistant en 2 à 15% d'au moins une méthylcellulose hydroxy propyle, 0,5 à 12% en poids d'au moins un alginate sodium, 0,1 à 10% en poids d'au moins une gomme guar ou mélange de ces derniers, et avec un sel de calcium acceptable pharmaceutiquement, optionnellement un diluant et un lubrifiant, pour former un mélange, et comprimer le mélange résultant en comprimé.

25. Procédé selon la revendication 24, **caractérisé en ce que** le mélange est mis à sec sous forme granulaire avant la compression.

26. Procédé selon la revendication 24, **caractérisé en ce que** le mélangé est mis sous forme granulaire humide avant la compression.

27. Composition selon l'une des revendications précédentes, sous la forme d'un comprimé.
